# EUROPEAN PATENT SPECIFICATION

(11) **EP 3 779 849 B1**
(45) Date of publication and mention of the grant of the patent: **15.01.2025**
(21) Application number: 19776863.3
(22) Date of filing: 27.03.2019
(51) Int. Cl.: G06Q 50/00, G06F 17/00, A61B 5/11, A01K 61/10

(54) **DEVICE AND METHOD FOR MONITORING ACTIVITY IN FISH**
VORRICHTUNG UND VERFAHREN ZUR ÜBERWACHUNG DER AKTIVITÄT IN FISCHEN
DISPOSITIF ET PROCÉDÉ POUR LA SURVEILLANCE DE L'ACTIVITÉ CHEZ DES POISSONS

(30) Priority: 28.03.2018 ES 201830305
(43) Date of publication of application: 17.02.2021
(73) Proprietor: Consejo Superior De Investigaciones Científicas, 28006 Madrid (ES); Universidad de Las Palmas de Gran Canaria, 35017 Las Palmas (ES)
(72) Inventor: CABRUJA CASAS, Enric, 28760 Tres Cantos (Madrid) (ES); LOZANO FANTOBA, Manuel, 08193 Cerdanyola del Vallès (Barcelona) (ES); PÉREZ SÁNCHEZ, Jaume, 12595 Cabanes (Cast. de la Plana) (ES); CALDUCH GINER, Josep, 12595 Cabanes (Cast. de la Plana) (ES); SOSA GONZÁLEZ, Carlos Javier, 35017 Las Palmas (ES); FERRER BALLESTER, Miguel Ángel, 35017 Las Palmas (ES); MONTIEL NELSON, Juan Antonio, 35017 Las Palmas (ES); AFONSO LÓPEZ, Juan Manuel, 35017 Las Palmas (ES)
(74) Representative: Pons
(86) International application number: PCT/ES2019/070205
(87) International publication number: WO 2019/185965

(56) References cited:
- WO-A1-01/08417
- WO-A1-01/08417
- CN-U- 201 897 919
- KR-A- 20130 005 484
- ERIKSON ULF ET AL: "Crowding of Atlantic salmon in net-pen before slaughter", AQUACULTURE, ELSEVIER, AMSTERDAM, NL, vol. 465, 16 September 2016 (2016-09-16), pages 395 - 400, XP029758478, ISSN: 0044-8486, DOI: 10.1016/J.AQUACULTURE.2016.09.018
- METCALFE J D; WRIGHT S; TUDORACHE C; WILSON R P: "Recent advances in telemetry for estimating the energy metabolism of wild fishes", JOURNAL OF FISH BIOLOGY, vol. 88, no. 1, 22 November 2015 (2015-11-22), pages 284 - 297, XP055745760, ISSN: 0022-1112, DOI: 10.1111/jfb.12804
- GUAN-ZHENG LIU; YAN-WEI GUO; QING-SONG ZHU; BANG-YU HUANG; LEI WANG: "Estimation of respiration rate from three-dimensional acceleration data based on body sensor network", TELEMEDICINE JOURNAL AND E-HEALTH, vol. 17, no. 9, November 2011 (2011-11-01), pages 705 - 711, XP055084098, ISSN: 1530-5627, DOI: 10.1089/tmj.2011.0022
- J KOLAREVIC , AAS-HANSSEN , A ESPMARK , G BAEVERFJORD , B FYHN TERJESEN , B DAMSGARD: "The use of acoustic acceleration transmitter tags for monitoring of Atlantic salmon swimming activity in recirculating aquaculture systems (RAS", AQUACULTURAL ENGINEERING, vol. 72, no. 73, 2016, pages 30 - 39, XP029639998, ISSN: 0144-8609, DOI: 10.1016/j.aquaeng.2016.03.002
- ERIKSON ULF; GANSEL LARS; FRANK KEVIN; SVENDSEN EIRIK; DIGRE HANNE: "Crowding of Atlantic salmon in net-pen before slaughter", AQUACULTURE, vol. 465, 2016, pages 395 - 400, XP029758478, ISSN: 0044-8486, DOI: 10.1016/j.aquaculture.2016.09.018
- D VAN DER LELIE , P CORBISIER ,W BAEYENS , S WUERTZ , L DIELS ,M MERGEAY: "The use of biosensors for environmental monitoring", RESEARCH IN MICROBIOLOGY, vol. 145, no. 1, 1994, pages 67 - 74, XP023925053, ISSN: 0923-2508, DOI: 10.1016/0923-2508(94)90073-6

## Description

### OBJECT OF THE INVENTION

The present invention belongs to the technical field of control and monitoring technologies of the well-being of animals, especially in aquaculture, by means of the monitoring of the physical activity and the breathing frequency.

A device for monitoring physical activity and breathing frequency in fish is described. The device is configured to be installed in the fish's operculum (or gill cover of the fish), and by means of an acceleration sensor, to record the accelerations thereof in the plane of the gill cover and in the normal direction thereof to said plane.

The method for monitoring the accelerations of the gill cover enables the breathing frequency of the fish and the movements resulting from the swimming activity to be extracted, jointly and simultaneously, in order to determine the metabolic state and well-being of the animal. The measurements obtained with the device may be complemented with measurements of environmental parameters of the facility or culture medium, obtained both in real time and predictively.

### BACKGROUND OF THE INVENTION

Aquaculture is the only animal production sector with a wide growth margin worldwide. Currently, aquaculture contributes 50% of the products derived from the sea, this contribution being expected to increase by about 80 million tons in the year 2050.

In the last decade, important advances have been made in the formulation of diets, diagnosis and disease prevention and genetic improvement programs. However, an urgent demand from the industry is the development and implementation of new biological monitoring systems for both the environment and the cultured animals. The ultimate goal is to remotely and non-invasively monitor the metabolic and/or health state of cultured animals. This practice is necessary for the development of new cultivation techniques that enable guaranteeing animal well-being while maximising production efficiency in a scenario of global change, with an increase in temperature, acidification of seawater and a decrease in the concentration of dissolved oxygen in the water.

The use of tags to identify or mark fish, as described in US4790090, has been a known method for more than 25 years. In their initial version, these tags were passive elements which simply identified the animal. These tags are usually attached to the body or to a fin. In document UA70087, tags that are fixed to the fish's operculum (or gill cover) are described, but they are passive tags without any electronic elements in them.

More recently electronic versions have appeared, in which the reading of the tag is carried out wirelessly by radio frequency, but they are also limited to identifying each animal with a unique code, as for example in those described in document US6400338.

One of the methods used for monitoring fish activity status is based on video camera recording and image analysis (document RU2596498). This method is complex and difficult to use routinely in the open sea or with high cultivation densities.

On the other hand, for years, miniaturised acceleration sensors have been used to study the activity of people. In the post *"*Activity recognition using cell phone accelerometers", Kwapisz et al. (2010) describe an algorithm for detecting the different activities of a person based on the acceleration sensor located in their pocket. The procedure is based on the acceleration to detect the type of movement carried out (running, walking, sitting, etc.). This algorithm is not compatible with the movement patterns of other living beings.

Similarly, document WO2015123373 refers to a system and method for detecting movement patterns in any type of object, animal or person by means of a device with a movement sensor that is attached to the individual. The system and method require classifying the type of movement according to a previously classified pattern. Classifying movement is not a sufficient condition to determine physical activity and breathing frequency, jointly and simultaneously, in fish.

In the post *"Accelerometer tags: detecting and identifying activities in fish and the effect of sampling frequency",* Broell *et al.* (2013) describe the use of implantable acceleration sensors in fish to monitor the activity thereof. More specifically, the publication refers to an algorithm for detecting the different activities of fish such as the moment of feeding or escape. They use the acceleration obtained from an acceleration sensor located on the first dorsal fin by means of a Velcro^{®}, without considering the breathing frequency.

A similar approach is described in *"*Recent advances in telemetry for estimating the energy metabolism of wild fishes"(Metcalfe et al., 2016) for evaluating the metabolic activity of fish by means of acceleration sensors, without considering the breathing frequency, and estimating the oxygen consumption based on the dynamics of the accelerations of the fish.

Finally, document WO01/08417A1 describes a system, method and apparatus for monitoring and/or displaying characteristics of a target. A remote unit is provided which is worn in a remote target's body or embedded in moving or standing objects. Each remote unit includes a data acquisition circuit and one or more internal sensors integrally formed on a self-contained application specific integrated circuit, or integrated circuit chipset. The system provides enhanced viewing capabilities by combining video images with collected data regarding the target.

### DESCRIPTION OF THE INVENTION

The present invention describes a device for controlling and monitoring a metabolic state, activity and well-being of fish according to claim 1, which allows autonomously, remotely and individually monitoring physical activity and breathing frequency. The present invention also describes a method for controlling and monitoring a metabolic state, activity and well-being of fish according to claim 7.

Thus, the present invention enables the technical problems of the state of the art of obtaining the breathing frequency of the fish, and the physical activity, jointly and simultaneously; and of monitoring the level of physical activity related to breathing frequency, swimming speed and oxygen consumption, to be solved.

The device comprises a substrate, for example flexible, preferably a tag, which in the simplest form thereof comprises: an acceleration sensor; a control unit (microcontroller) configured to partially or completely execute an activity-monitoring routine; a memory; a battery; an external measurement unit for downloading data, and optionally for processing thereof; optionally, a transmitter-receiver for optical, electrical, magnetic or electromagnetic communication between the substrate attached to the fish's operculum (or gill cover of the fish) and the external unit.

The acceleration sensor is an inertial device which provides acceleration data in three orthogonal axes, called x, y, z; and which enables the detection of small movements of the gill cover and the displacement of the fish, in the form of accelerations. The accelerations are stored in the memory and processed, completely or partially, by the microcontroller in order to quantify the breathing frequency and the level of physical activity.

The microcontroller provides the computing power for executing, completely or partially, the activity-monitoring routine that enables the calculation of physical activity and breathing frequency. For this, it has multiple modules that enable the control, storage, processing, and communication of the data generated by the acceleration sensor. The microcontroller provides a communication interface with the external unit that enables the programming, configuration and download of the data generated by the acceleration sensor. Likewise, it provides an internal interface to communicate with said acceleration sensor.

The memory is preferably non-volatile and is used to store the activity-monitoring routine, the acquired accelerations and the data resulting from the analysis of the physical activity and breathing frequency. The microcontroller has a power management unit to minimise battery consumption during periods of inactivity. The microcontroller has an event planning unit for the temporal execution of the experiments (data collection from the acceleration sensor and complete or partial processing). Events can be programmed in the memory or triggered from the external unit.

The battery is, in an exemplary embodiment, rechargeable and high-performance to provide enough energy to the rest of the components of the electronic circuit (acceleration sensor, microcontroller, memory, and the transmitter-receiver when present) during the useful life of the device.

The substrate is temporarily or permanently located in the gill cover of the fish to be monitored. Implantation in the gill cover can be done for example by means of perforations for the fixing thereof, or by means of a clamp mechanism, or by means of an adhesive substance. By means of the acceleration sensor, the movements of the fish while swimming, as well as the movements of the gill cover, jointly and simultaneously, are recorded in the form of accelerations. Both movements, which are fused into a single multidimensional signal, are separated by processing the data acquired by the acceleration sensor and are used to determine the swimming activity and the frequency at which the fish breathes. The data obtained by the acceleration sensor located in the gill cover is stored in memory and, subsequently, completely or partially processed by the control unit. In order to read the data from the device, for example, the fish is captured, the substrate is removed and the information stored in memory is downloaded onto a computer by means of the external drive; or, without removing the device, the data is downloaded by means of optical, electrical, magnetic, or electromagnetic communication.

The activity-monitoring routine is based on the calculation of the gill cover frequency in the direction that is normal to the plane of the gill cover, z axis. In addition, a statistical calculation of the accelerations that are coplanar to the gill cover, xy plane, is carried out to obtain an indicator of fish activity.

If the fish shows symptoms of stress, malnutrition or disease, among others, it will breathe at an abnormal frequency, and the behaviour thereof may be affected by erratic movements of high activity or a rest state. After a behaviour study, which is specific to each species, culture condition and development phase, it is possible to establish a comfort function, such that based on the acceleration sensor readings, the breathing frequency and physical activity having been extracted, the metabolic state and degree of well-being of the animal is determined. This determination may eventually be refined by means of readings of environmental parameters, obtained both in real time and predictively by means of other sensors.

As previously described, there are commercial tags for identifying fish, but in all cases they are passive and only transmit an identification code of the animal. There are also research groups which work with acceleration sensors in fish, but they only record patterns of swimming movement, without taking into account the breathing frequency in any case. Thanks to the device of the present invention, which records swimming and breathing movements, jointly and simultaneously, with a single sensor device, reliable measurements of the degree of well-being of the fish can be obtained.

In an exemplary embodiment, the components of the electronic circuit are mounted on the preferably flexible substrate by means of encapsulation techniques such as, for example, inverted circuit technology, wire soldering or surface mounting. Also in an exemplary embodiment, the water-resistamt (or impervious) coating is made of insulating epoxy to prevent water contact with the components of the electronic circuit.

In an exemplary embodiment, the control unit is configured to completely execute an activity-monitoring routine comprising filtering and analysing the multidimensional signal obtained by the acceleration sensor. The analysis determines the breathing frequency and physical activity of the fish. The external unit downloads the data obtained in the analysis.

In an exemplary embodiment, the control unit is configured to partially execute an activity-monitoring routine. In the external processing unit, after downloading the data, these are filtered and processed to determine the breathing frequency and physical activity.

In another exemplary embodiment, the control unit is configured to acquire the accelerations that are stored in memory. In the external processing unit, after downloading the data, these are filtered and processed to determine the breathing frequency and physical activity.

### DESCRIPTION OF THE DRAWINGS

To complement the description that is being made and for the purpose of helping to better understand the features of the invention according to a preferred practical exemplary embodiment thereof, a set of drawings is attached as an integral part of said description in which the following is depicted in an illustrative and non-limiting manner:
Figure 1 shows a device implanted in the fish's operculum.
Figure 2 shows the electronic circuit of the device, the accelerometer, the battery, and an interface of an external device has been represented to show how communication is carried out (sending data, programming) between the substrate and the external device.
Figure 3 shows a diagram which describes the method for monitoring the activity of the fish by means of the breathing frequency and the activity rate thereof.

### PREFERRED EMBODIMENT OF THE INVENTION

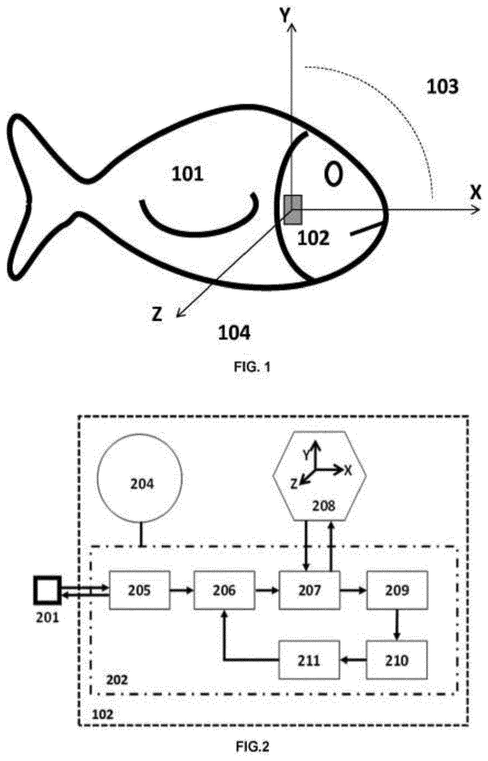

Below, an exemplary embodiment of the present invention is described with the aid of figures 1 to 3.

The substrate of the device for monitoring fish of the present invention is configured to be placed on the fish's operculum (or gill cover of a fish) to determine different types of physical activity and breathing frequency. Figure 1 shows the substrate of the device (102) already placed on the gill cover of a fish (101) and the three orthogonal axes (x, y, z) in which the accelerations are measured have been represented. For the monitoring, the acceleration measurements in the xy plane (103) are used, which is coplanar to the gill cover, from which the swimming movement of the fish is extracted; and, in addition, the measurements of acceleration in the z axis (104), which is normal to the gill cover, from which the breathing frequency of the fish is obtained based on the movements of the gill cover.

Figure 2 shows the electronic circuit diagram of the device (102). This electronic circuit is installed on a flexible substrate and protected from water by an impervious coating.

The electronic circuit comprises:
a) an acceleration sensor (208) intended for acquiring data on the accelerations of the fish to which the device is attached in the plane of the gill cover, xy (103) and in the direction which is normal to the gill cover, z-axis (104);
b) a control unit (202), preferably a microcontroller, which has all the necessary subsystems: communication, serial bus, program and data memory, energy consumption control system, among others. This control unit is connected to the acceleration sensor (208); and is configured to receive data from said acceleration sensor, for example, by means of I2C interface of the bus (207), to store it for example in a non-volatile memory (210) and to transmit it to an external device (201). The control unit (202) is configured to execute at least one monitoring routine of the physical activity and of the breathing frequency (209);
c) a battery (204) that supplies the acceleration sensor (208) and the control unit (202) with all the subsystems thereof.

In an exemplary embodiment of the invention, the control unit (202) includes a communication interface (205) with an external unit (201). This communication is carried out by means of an optical, electrical, magnetic and/or electromagnetic transmitter/receiver. In the external unit (201) the activity-monitoring routine is executed, completely or partially.

The interface (205) enables the programming of the control unit (202) by the external unit (201).

The control unit (202) may further be configured to execute, in a microprocessor (206), completely or partially, the activity-monitoring routine. The control unit (202) further executes an experiment-planning routine. This routine enables the timing and synchronisation of the data collection of the acceleration sensor (208) to be controlled during a determined period of time.

Likewise, the control unit includes an energy manager (211) to reduce energy consumption during periods of data collection. The subsystem for computing the acquired acceleration data (209) comprises a data analysis routine to determine the breathing frequency and the physical activity.

Preferably the battery (204) is of minimal size and weight but high performance to allow long periods of execution of the data-analysis routine.

In a preferred embodiment of the invention, the electronic circuit is arranged in an encapsulation that is as small as possible to minimise the weight and size of the device.

In a preferred embodiment, the electronic components are surface-mounted, and are chosen with the minimum size. The battery can be button-type, rechargeable with a minimum diameter. The support substrate for the entire system is preferably made of a flexible organic material to allow one of the ends thereof to bend on itself in order to connect the button-type battery on both sides.

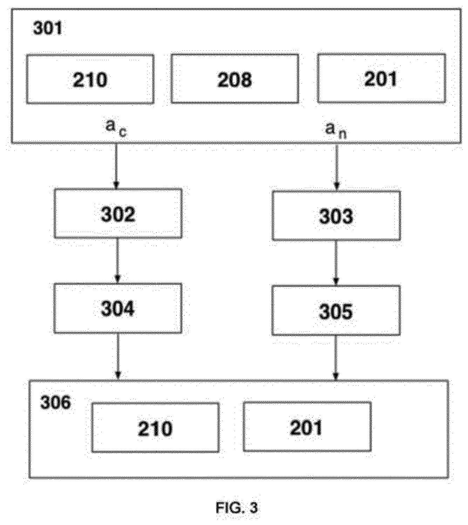

Figure 3 shows the different stages of the activity-monitoring routine that includes the procedure for calculating physical activity and breathing frequency in fish proposed in the present invention. The first necessary step is to obtain the acceleration data (301) in the three orthogonal axes x, y, z (aₓ, a_{y}, a_{z}) either from the accelerometer (208), from the memory (210) or from the external unit (201).

The a_{c} accelerations that are coplanar to the gill cover are contained in the xy plane, and the aₙ acceleration that is normal to the gill cover corresponds to the z axis. These data are obtained with the acceleration sensor (208) or are stored in the memory (210) after being acquired, or are available in the external unit (201).

In an exemplary embodiment, the mean value (303) is subtracted from the acceleration data in the aₙ direction that is normal to the gill cover, for example, the z-axis accelerations (a_{z}), and, subsequently, a count is made of the number and distance of maximums, and thus the breathing frequency (305) is determined. Likewise, the mean value is also subtracted from the a_{c} coplanar acceleration data, for example, the x-axis (aₓ) and y-axis (a_{y}) accelerations, respectively (302). From the results obtained, a physical activity rate (304) is determined.

Finally, a step of storing (306) in the memory (210) or in the external unit (201), the data obtained from the breathing frequency (305) and the activity rate (304) is carried out.

A certain time interval is selected and during intervals of said times, representative values are calculated for each set of samples of the acceleration sensor obtained on each axis with a sampling frequency.

## Claims

1. A device for controlling and monitoring a metabolic state, activity and well-being of fish, intended to be attached to a fish's operculum, wherein the device comprises a substrate (102) with an electronic circuit and a waterresistant coating that covers the electronic circuit, the device being configured to join temporarily or permanently to the fish's operculum, of which the physical activity and breathing frequency is to be controlled and monitored during periods of ingestion and non-feeding throughout the day, wherein the electronic circuit comprises:
- a three orthogonal-axis (x, y, z) acceleration sensor (208) configured to acquire coplanar and normal accelerations of the fish's operculum jointly and simultaneously,
- a control unit (202) connected to the acceleration sensor (208) and which contains at least one microcontroller (206) configured to process the coplanar and normal accelerations obtained by the three orthogonal-axis (x, y, z) acceleration sensor (208), separating swimming movements and movements of gill cover to determine swimming activity from the accelerations in the z (a_{z}) axis which is normal to the fish's operculum and a frequency at which the fish breathes from the accelerations in x, y (aₓ, a_{y}) axes which are coplanar to the fish's operculum, by executing at least one activity-monitoring routine,
- a memory (210) configured to store data acquired by the acceleration sensor (208) and/or data generated by the microcontroller (206) when executing an activity-monitoring routine, and
- a battery (204) connected to the control unit (202),
wherein the substrate (102) is configured to establish communication with an external device (201) to which the data acquired by the acceleration sensor (208) and/or the data generated by the microcontroller (206) are sent.

2. The device for controlling and monitoring the metabolic state, activity and well-being according to claim 1, **characterised in that** the control unit (202) comprises a communication interface (205) configured to establish communication with the external device (201) to send the data obtained by the acceleration sensor (208) and/or data stored in the memory (210).

3. The device for controlling and monitoring the metabolic state, activity and well-being of fish according to claim 1, **characterised in that** the control unit (202) comprises a communication bus (207) linked to the acceleration sensor (208) and configured to establish the communication with said acceleration sensor (208).

4. The device for controlling and monitoring the metabolic state, activity and well-being of fish according to claim 1, **characterised in that** additionally comprises an energy manager (211) linked to the microcontroller (206) and to the battery (204) and configured to control resting times and data collection times during the execution of the activity-monitoring routine.

5. The device for controlling and monitoring the metabolic state, activity and well-being of fish according to claim 1, **characterised in that** it additionally comprises an external unit (201) configured to receive and process the data sent by the control unit (202).

6. The device for controlling and monitoring the metabolic state, activity and well-being of fish according to claim 1, **characterised in that** the control unit (202) comprises at least one data computing chip (209) configured to process the data from the acceleration sensor (208).

7. A method for controlling and monitoring a the- metabolic state, activity and well-being of fish with a device intended to be attached to a fish's operculum, for controlling and monitoring a metabolic state, activity and well-being of fish as described in any one of claims 1 to 6, said method comprising attaching said device to the fish's operculum and executing a monitoring routine wherein the following steps are performed:
- measuring with the acceleration sensor (208) accelerations in three orthogonal axes, two being coplanar to the fish's operculum x, y, (*aₓ, a_{y}*) axis, and a third being normal to the fish's operculum z (*a_{z}*) axis, jointly and simultaneously;
- calculating in predetermined time intervals, representative values of a plurality of samples of the acceleration sensor (208) obtained in each x, y, z axis with a predetermined sampling frequency;
- extracting breathing frequency from the accelerations in the z (a_{z}) axis;
- calculating physical activity rate from the accelerations in the x, y (*aₓ a_{y}*) axes.

## Patentansprüche

1. Vorrichtung zum Steuern und Überwachen eines Stoffwechselzustands, einer Aktivität und eines Wohlbefindens von Fischen, die dazu bestimmt ist, an einem Kiemendeckel eines Fisches angebracht zu werden, wobei die Vorrichtung ein Substrat (102) mit einer elektronischen Schaltung und einer wasserfesten Beschichtung umfasst, die die elektronische Schaltung bedeckt, wobei die Vorrichtung dazu konfiguriert ist, vorübergehend oder permanent mit dem Kiemendeckel des Fisches verbunden zu werden, dessen körperliche Aktivität und Atemfrequenz während Perioden der Nahrungsaufnahme und des Nicht-Fütterns im Laufe des Tages zu steuern und zu überwachen ist, wobei die elektronische Schaltung Folgendes umfasst:
- einen Beschleunigungssensor (208) für drei orthogonale Achsen (x, y, z), der dazu konfiguriert ist, koplanare und normale Beschleunigungen des Kiemendeckels des Fisches gemeinsam und gleichzeitig zu erfassen,
- eine Steuereinheit (202), die mit dem Beschleunigungssensor (208) verbunden ist und mindestens einen Mikrocontroller (206) enthält, der dazu konfiguriert ist, die koplanaren und normalen Beschleunigungen zu verarbeiten, die durch den Beschleunigungssensor (208) für drei orthogonale Achsen (x, y, z) erhalten werden, wobei Schwimmbewegungen und Bewegungen des Operculums getrennt werden, um die Schwimmaktivität aus den Beschleunigungen in der z-Achse (a_{z}), die normal zum Kiemendeckel des Fisches ist, und eine Frequenz, mit der der Fisch atmet, aus den Beschleunigungen in den x-, y-Achsen (aₓ, a_{y}), die koplanar zum Kiemendeckel des Fisches sind, zu bestimmen, indem mindestens eine Aktivitätsüberwachungsroutine ausgeführt wird,
- einen Speicher (210), der dazu konfiguriert ist, Daten, die durch den Beschleunigungssensor (208) erfasst werden, und/oder Daten, die durch den Mikrocontroller (206) generiert werden, wenn eine Aktivitätsüberwachungsroutine ausgeführt wird, zu speichern, und - eine Batterie (204), die mit der Steuereinheit ( 202) verbunden ist, wobei das Substrat (102) dazu konfiguriert ist, eine Kommunikation mit einer externen Vorrichtung (201) herzustellen, an die die durch den Beschleunigungssensor (208) erfassten Daten und/oder die durch den Mikrocontroller (206) generierten Daten gesendet werden.

2. Vorrichtung zum Steuern und Überwachen des Stoffwechselzustands, der Aktivität und des Wohlbefindens nach Anspruch 1, **dadurch gekennzeichnet, dass** die Steuereinheit (202) eine Kommunikationsschnittstelle (205) umfasst, die dazu konfiguriert ist, eine Kommunikation mit der externen Vorrichtung (201) herzustellen, um die durch den Beschleunigungssensor (208) erhaltenen Daten und/oder im Speicher (210) gespeicherte Daten zu senden.

3. Vorrichtung zum Steuern und Überwachen des Stoffwechselzustands, der Aktivität und des Wohlbefindens nach Anspruch 1, **dadurch gekennzeichnet, dass** die Steuereinheit (202) einen Kommunikationsbus (207) umfasst, der mit dem Beschleunigungssensor (208) verknüpft und dazu konfiguriert ist, die Kommunikation mit dem Beschleunigungssensor (208) herzustellen.

4. Vorrichtung zum Steuern und Überwachen des Stoffwechselzustands, der Aktivität und des Wohlbefindens von Fischen nach Anspruch 1, **dadurch gekennzeichnet, dass** sie zusätzlich einen Energiemanager (211) umfasst, der mit dem Mikrocontroller (206) und der Batterie (204) verknüpft und dazu konfiguriert ist, Ruhezeiten und Datensammlungszeiten während der Ausführung der Aktivitätsüberwachungsroutine zu steuern.

5. Vorrichtung zum Steuern und Überwachen des Stoffwechselzustands, der Aktivität und des Wohlbefindens von Fischen nach Anspruch 1, **dadurch gekennzeichnet, dass** sie zusätzlich eine externe Einheit (201) umfasst, die dazu konfiguriert ist, die durch die Steuereinheit (202) gesendeten Daten zu empfangen und zu verarbeiten.

6. Vorrichtung zum Steuern und Überwachen des Stoffwechselzustands, der Aktivität und des Wohlbefindens von Fischen nach Anspruch 1, **dadurch gekennzeichnet, dass** die Steuereinheit (202) mindestens einen Datenrechenchip (209) umfasst, der dazu konfiguriert ist, die Daten des Beschleunigungssensors (208) zu verarbeiten.

7. Verfahren zum Steuern und Überwachen eines Stoffwechselzustands, einer Aktivität und eines Wohlbefindens von Fischen mit einer Vorrichtung, die dazu bestimmt ist, an einem Kiemendeckel eines Fisches angebracht zu werden, zum Steuern und Überwachen eines Stoffwechselzustands, einer Aktivität und eines Wohlbefindens von Fischen, wie in einem der Ansprüche 1 bis 6 beschrieben, wobei das Verfahren das Anbringen der Vorrichtung an dem Kiemendeckel eines Fisches und das Ausführen einer Überwachungsroutine umfasst, wobei die folgenden Schritte durchgeführt werden:
- Messen von Beschleunigungen in drei orthogonalen Achsen mit dem Beschleunigungssensor (208), wobei zwei koplanar zum Kiemendeckel des Fisches (*aₓ,* a_{y}), x-, y-Achsen, sind und eine dritte normal zum Kiemendeckel des Fisches (a_{z}), z-Achse, ist, gemeinsam und gleichzeitig;
- Berechnen von repräsentativen Werten einer Vielzahl von Proben des Beschleunigungssensors (208), die in jeder x-, y- und z-Achse mit einer vorbestimmten Abtastfrequenz erhalten werden, in vorbestimmten Zeitintervallen;
- Extrahieren der Atemfrequenz aus den Beschleunigungen in der z-Achse (aZ);
- Berechnen der körperlichen Aktivitätsrate aus den Beschleunigungen in den x-, y-Achsen (*aₓ, a_{y}*)*.*

## Revendications

1. Dispositif permettant de contrôler et surveiller un état métabolique, l'activité et le bien-être de poissons, destiné à être fixé à l'opercule d'un poisson, dans lequel le dispositif comprend un substrat (102) doté d'un circuit électronique et d'un revêtement résistant à l'eau qui recouvre le circuit électronique, le dispositif étant conçu pour s'attacher temporairement ou définitivement à l'opercule du poisson, dont l'activité physique et la fréquence respiratoire doivent être contrôlées et surveillées pendant des périodes d'ingestion et de nonalimentation tout au long de la journée, dans lequel le circuit électronique comprend :
- un capteur d'accélération (208) selon trois axes orthogonaux (x, y, z) configuré pour acquérir conjointement et simultanément des accélérations coplanaires et normales de l'opercule du poisson,
- une unité de contrôle (202) connectée au capteur d'accélération (208) et qui contient au moins un microcontrôleur (206) configuré pour traiter les accélérations coplanaires et normales obtenues par le capteur d'accélération (208) selon trois axes orthogonaux (x, y, z), séparant les mouvements de nage et les mouvements de l'opercule des branchies pour déterminer l'activité de nage à partir des accélérations selon l'axe z (a_{z}) qui est normal à l'opercule du poisson et une fréquence à laquelle le poisson respire à partir des accélérations selon les axes x, y (aₓ, a_{y}) qui sont coplanaires à l'opercule du poisson, en exécutant au moins une routine de surveillance d'activité,
- une mémoire (210) configurée pour stocker des données acquises par le capteur d'accélération (208) et/ou des données générées par le microcontrôleur (206) lors de l'exécution d'une routine de surveillance d'activité et - une batterie (204) connectée à l'unité de contrôle (202), dans lequel le substrat (102) est configuré pour établir une communication avec un dispositif externe (201) auquel les données acquises par le capteur d'accélération (208) et/ou les données générées par le microcontrôleur (206) sont envoyées.

2. Dispositif permettant de contrôler et surveiller l'état métabolique, l'activité et le bien-être selon la revendication 1, **caractérisé en ce que** l'unité de contrôle (202) comprend une interface de communication (205) configurée pour établir une communication avec le dispositif externe (201) pour envoyer les données obtenues par le capteur d'accélération (208) et/ou les données stockées dans la mémoire (210).

3. Dispositif permettant de contrôler et surveiller l'état métabolique, l'activité et le bien-être de poissons selon la revendication 1, **caractérisé en ce que** l'unité de contrôle (202) comprend un bus de communication (207) relié au capteur d'accélération (208) et configuré pour établir la communication avec ledit capteur d'accélération (208).

4. Dispositif permettant de contrôler et surveiller l'état métabolique, l'activité et le bien-être de poissons selon la revendication 1, **caractérisé en ce qu'**il comprend de plus un dispositif de gestion de l'énergie (211) relié au microcontrôleur (206) et à la batterie (204) et configuré pour contrôler les temps de repos et les temps de collecte de données pendant l'exécution de la routine de surveillance d'activité.

5. Dispositif permettant de contrôler et surveiller l'état métabolique, l'activité et le bien-être de poissons selon la revendication 1, **caractérisé en ce qu'**il comprend de plus une unité externe (201) configurée pour recevoir et traiter les données envoyées par l'unité de contrôle (202).

6. Dispositif permettant de contrôler et surveiller l'état métabolique, l'activité et le bien-être de poissons selon la revendication 1, **caractérisé en ce que** l'unité de contrôle (202) comprend au moins une puce de calcul informatique de données (209) configurée pour traiter les données provenant du capteur d'accélération (208).

7. Procédé permettant de contrôler et surveiller un état métabolique, l'activité et le bien-être de poissons avec un dispositif destiné à être fixé à l'opercule d'un poisson, permettant de contrôler et surveiller un état métabolique, l'activité et le bien-être de poissons tel que décrit dans l'une quelconque des revendications 1 à 6, ledit procédé comprenant la fixation dudit dispositif à l'opercule du poisson et l'exécution d'une routine de surveillance dans laquelle les étapes suivantes sont effectuées :
- la mesure avec le capteur d'accélération (208) d'accélérations selon trois axes orthogonaux, deux étant coplanaires à l'opercule du poisson (*aₓ,* a_{y}), axes x, y, et une troisième étant normale à l'opercule du poisson (a_{z}), axe z, conjointement et simultanément ;
- le calcul à des intervalles de temps prédéterminés, de valeurs représentatives d'une pluralité d'échantillons du capteur d'accélération (208) obtenus selon chaque axe x, y, z avec une fréquence d'échantillonnage prédéterminée ;
- l'extraction de la fréquence respiratoire à partir des accélérations selon l'axe z (aZ) ;
- le calcul du taux d'activité physique à partir des accélérations selon les axes x, y (*aₓ a_{y}*)*.*
